# EUROPEAN PATENT SPECIFICATION

(11) **EP 1 562 621 B1**
(45) Date of publication and mention of the grant of the patent: **19.08.2009**
(21) Application number: 03777001.3
(22) Date of filing: 20.11.2003
(51) Int. Cl.: A61K 38/00, A61P 17/00

(54) **COMPOSITION FOR TREATING ICHTHYOSIS USING ANTITRYPSIN**
ZUSAMMENSETZUNG ZUR BEHANDLUNG VON ICHTHYOSIS UNTER VERWENDUNG VON ANTITRYPSIN
COMPOSITION POUR LE TRAITEMENT DE L'ICHTYOSE UTILISANT L'ANTITRYPSINE

(30) Priority: 20.11.2002 US 427702 P
(43) Date of publication of application: 17.08.2005
(73) Proprietor: Arriva-Prometic Inc., Mont-Royal QC H4P 2LP (CA)
(72) Inventor: BATHURST, Ian C., Alameda, CA 94501 (US); PEMBERTON, Philip A., Alameda, CA 94501 (US); SUNDIN, David J., Alameda, CA 94501 (US); MAYHEW, James W., Petaluma, CA 94954-1169 (US); ANGEL, Arturo J., Petaluma, CA 94954-1169 (US); BARR, Philip J., Alameda, CA 94501 (US)
(74) Representative: Perry, Robert Edward
(86) International application number: PCT/GB2003/005049
(87) International publication number: WO 2004/045634

(56) References cited:
- EP-A- 0 420 600
- WO-A-00/07620
- WO-A-01/30380
- WO-A-01/64132
- WO-A-92/06706
- WO-A-99/02665
- WO-A-99/49887
- GB-A- 2 318 732
- DATABASE WPI Section Ch, Week 198242 Derwent Publications Ltd., London, GB; Class A96, AN 1982-88806E XP002277044 & JP 57 145817 A (YAMAZAKI T), 9 September 1982 (1982-09-09)

## Description

### TECHNICAL FIELD

This invention relates to a protease inhibitor composition and use of the composition for the manufacture of a medicament for therapeutic applications. In particular, the invention relates to a α1-antitrypsin composition for the manufacture of a medicament for the treatment or prevention of ichthyosis.

### BACKGROUND OF THE INVENTION

Hyperproliferative and inflammatory skin or mucocutaneous disorders affect millions of individuals in the United States every year. Such disorders range from mild to life threatening and include, for example, skin cancer, atopic dermatitis, psoriasis, and asthma due to the inflammation of the lung mucosa. In addition, extrinsic skin aging can be caused by chronic inflammation and insufficient skin repair due to repetitive exposures to environmental insults, *e*.*g*. ultraviolet radiation.

Atopic dermatitis is very common in all parts of the world. This chronically relapsing inflammatory skin disorder affects about ten percent of infants and three percent of the U.S. population overall. The disease can occur at any age, but is most common in infants to young adults (*see*, Hanifin, et al, Arch. Dermatol, 135(12):1551 (1999)). The face is often affected first, then the hands and feet. Sometimes dry red patches appear all over the body. In older children, the skin folds are most often affected, especially the elbow creases and behind the knees. In adults the face and hands are more likely to be involved.

Eczema is another example of a common inflammatory disorder. Eczema is a red, itchy, noncontagious inflammation of the skin that may be acute or chronic, with red skin patches, pimples, crusts, or scabs occurring either alone or in combination. The skin may be dry, or it may discharge a watery fluid, resulting in an itching or burning sensation. The affected skin may become infected. The various causes of eczematous dermatitis are classified as either external (irritations, allergic reactions, exposure to certain microorganisms or chemicals, etc.), congenital (inherited predisposition) and environmental (stress, heat, etc). Eczema may clear for years, only to reappear later at a different site. Eczema can come in any of several forms, including, most commonly, atopic dermatitis.

A condition similar to atopic dermatitis, but which affects mucosal tissues rather than the skin, is asthma. Asthma is a chronic lung disease characterized by inflammation of the air passages. This condition is estimated to affect about 15 million Americans and can be severe and result in death if not treated. A number of factors can exacerbate asthma including, *e*.*g*., rapid changes in temperature or humidity, allergies, upper respiratory infections, exercise, or stress. Typical treatments include bronchodilators which are given orally or delivered as an aerosol (inhaled) and, for the most difficult cases, corticosteroids. Another example of a mucocutaneous inflammatory disorder is allergic rhinitis (hay fever). Allergic rhinitis is caused by a nasal inflammation in response to an irritant or an allergen. This condition can be seasonal or occur throughout the year (perennial). Currently, allergic rhinitis is treated by the administration of antihistamines either orally or locally (*i*.*e*., using nasal sprays).

Other examples of mucocutaneous inflammatory disorders include those that involve comification and papulosquamous disorders. Examples of such disorders include lamellar ichthyosis, acne, rosacea, psoriasis, and lichen planus. Papulosquamous disorders are those characterized, as the name suggests, by scaly papules and plaques. Some of the more common papulosquamous disorders include psoriasis and lichen planus, both of which are manifested by a local inflammation of either the skin or a mucosal tissue (*e*.*g*., in the case of oral lichen planus).

Psoriasis is a persistent skin disease. The skin becomes inflamed, producing red, thickened areas with silvery scales, most often on the scalp, elbows, knees, and lower back. Severe psoriasis may cover large areas of the body. Psoriasis is not contagious, and has some genetic basis as it is more likely to occur in people whose family members have it. In the United States about 2% of adults have psoriasis (four to five million people). Approximately 150,000 new cases occur each year. The cause of psoriasis is unknown, however, recent discoveries point to an abnormality in the functioning of key white cells in the blood stream triggering inflammation in the skin. Psoriasis is thus thought to be due, at least in part, to an abnormal immune reaction against some component of the skin. This leads to the local infiltration of inflammatory cells, including leukocytes, into the tissues, expression of cell adhesion molecules, and the up-regulation of inflammatory cytokines and growth factors. As a result, the two hallmark features of psoriasis are local inflammation and epidermal hyperproliferation. The combination of hyperproliferation with incomplete terminal differentiation leads to the formation of a thickened *stratum corneum* or plaques.

Hyperproliferative skin disorders result from the loss of the regulatory mechanisms that control the proliferation and differentiation of skin cells. Basal and squamous cell carcinomas are the most common forms of skin cancer. About 1.3 million cases of skin carcinomas are found in the United States per year. Both basal and squamous cell carcinoma (*i*.*e*. Kaposi's sarcoma) affect the most external layer of the skin, the epidermis, and begin at the basal cell layer and at the upper cell layer of the epidermis, respectively. Although these skin carcinomas are slow growing and usually benign, they can, if not treated, grow and invade other tissues.

In addition to changes resulting from inflammatory and hyperproliferative disorders, the appearance and characteristics of the skin also change as the body ages. Chronologically aged (intrinsically aged) mucocutaneous surfaces show a slight atrophy of the epidermis and weakening the dermal/epidermal junction. Dryness of the skin is a common phenomenon. The dermis shows a decrease in cell numbers and elastic fibers and thus, a reduction in skin elasticity. Capillaries are also fragile as evidenced by bruisability. Collagen metabolism is slower, and there is a progressive lowering in concentration of glycosaminoglycans, thus sagging of the skin occurs.

There is a decreased ability to mount inflammatory responses in aged skin and an increase in the time of healing after injury. Aging is accelerated in those areas exposed to environmental insults, such as, *i*.*e*., irritating substances and sunlight (ultraviolet radiation), due to the development of local skin inflammation. The skin aging process resulting from exposure to sunlight is known as "photoaging." Photoaging accounts for about 80% of the visible changes of skin aging. It induces deep wrinkles not erased by stretching, pigmentary alterations with areas of hyper- and hypo-pigmentation (actinic lentigines and leukodermas), and a variety of benign, premalignant, and malignant neoplasms. The dermis shows evidence of chronic inflammation with increased cellularity and enlarged fibroblasts.

While certain treatments have been developed for some of these conditions, the treatments are often ineffective, not tolerated by certain individuals, or associated with one or more side effects that limit their use. With some of these conditions, no effective treatments currently exist. The side effects exhibited by currently available treatments include a rebound of the disease activity upon withdrawal of medication (*i*.*e*., glucocorticoids, cyclosporin A-like drugs), an increase in the incidence of cancer (*i*.*e*., PUVA), and toxicity (*i*.*e*., antimetabolites, such as methotrexate). Additionally, certain procedures are extremely inconvenient (*i*.*e*., coal tar treatments) or invasive (*i*.*e*., surgery). Current treatments for skin photodamage include retinoids and alpha-hydroxy-acids that exhibit light sensitivity, limited efficacy, and untoward side effects. Excipients that are safe and contain active ingredients are rare and in high demand by the cosmeceutical industry.

Currently, there is a largely unmet medical need for effective and safe compositions for the treatment of inflammatory mucocutaneous disease or disorders. Topical formulations are typically preferred over the orally delivered drug in order to avoid adverse systemic side effects. Nevertheless, developing a suitable topical formulation for clinical use in the treatment of inflammatory mucocutaneous disease or disorders poses considerable challenges. Particularly, problems associated with sufficient penetration through the skin or mucous membrane remain a concern.

Additionally, in topical formulations containing a gel or gelling agent, problems with pH, solubility, consistency, and sterility of the gel arise. Achieving a uniform pH in a gel formulation is required for safety in topical products but is difficult to obtain. Furthermore, achieving sterility of a gel formulation may be difficult if filter sterilization and heat sterilization techniques cannot be used without destroying the activity of the active ingredient. In addition, suitable topical gels must have a solubility and consistency that allow spreading the gel over the area to be treated without losing contact with the site of injury.

In view of the foregoing, it is readily apparent that there is a great need in the art for new protease inhibitor composition for effective treatments of a large number of inflammatory and hyperproliferative mucocutaneous disorders. The present invention addresses these and other needs.

### SUMMARY OF THE INVENTION

According to the present invention, α1-antitrypsin and a gelling agent are used for the manufacture of a medicament for treating or preventing ichthyosis.

### DESCRIPTION OF THE INVENTION

The medicament (or pharmaceutical composition) is pharmaceutically acceptable. The term "pharmaceutically acceptable" as used herein means that the substance is approved by a regulatory agency of the Federal or a state government or listed in the U.S. Pharmacopoeia or other generally recognised pharmacopoeia for use in animals, and more particularly in humans.

The α1-antitrypsin used in the pharmaceutical composition may be a natural, isolated, synthetic or recombinant protein. The preferred concentration of α1-antitrypsin in the composition is 0.001% to 20% w/w, more preferably 0.1% to 3% w/w, and most preferably 1% to 1.5% w/w.

A preferred pharmaceutical composition for use in the invention contains α1-antitrypsin, a physiological buffer, and a gelling agent. The composition also optionally contains water. The most appropriate physiological buffers include tris, histidine, triethanolamine and salts. Salts are preferably NaCl, KCl or phosphate salts. The physiological buffer preferably contains 0-250 mM phosphate, 0-250 mM NaCl and 0-250 mM KCl. In a more preferred formulation, the physiological buffer contains 5-100 mM phosphate, 5-100 mM NaCl; and 5-100 mM KCl. The pH range of the physiological buffer used in the pharmaceutical compositions of the invention is preferably within the range of from about pH 6.0 to about 9.0, more preferably between about pH 6.5 to about 8.0, and most preferably between about pH 7.0 to about 7.5.

The gelling agent of the pharmaceutical composition incudes any pharmaceutically suitable gelling agents such as, for example, hydroxyethyl celluloses, poloxamers, hydroxypropyl celluloses (HPC), polyacrylic acids, polyoxyethylene-polyoxypropylene block copolymers, or any combination thereof. The concentration range of the gelling agent in the composition is, for example, from about 0.1% to about 50% w/w, preferably from about 0.1% to about 10% w/w, from about 0.5% to about 5% w/w, from about 1.5% to about 4% w/w, or from about 0.25% to about 2% w/w, or most preferably from about 0.3% to about 1% w/w.

The preferred gelling agent is a polyacrylic acid having a concentration from about 0.25% to about 2 % w/w or from about 0.3% to about 1% w/w of the composition. The pH of the polyacrylic acid gel is, for example, within the range of from about 5.0 to about 9.0, preferably between about 6.0 to about 8.0, and more preferably between about 6.5 to about 7.4. Polyacrylic acid polymer is also known as carbomer. A preferred polyacrylic acid polymer is sold under the trademark Carbopol^{™} polymer (Noveon, Inc., Cleveland, OH). The preferred grade of Carpobol ^{™} carbomer is P-934, or P-980.

Alternatively, the gelling agent is a polyoxyethylene-polyoxypropylene block copolymer in a concentration of from about 18% to about 35% w/w in the composition. Preferably, the concentration of the polyoxyethylene-polyoxypropylene block copolymer is from about 18% to about 25% w/w. The pH of the block copolymer gel is, for example, within the range of from about 5.0 to about 9.0, preferably between about 6.0 to about 8.0, and more preferably between about 6.5 to about 7.4. A preferred polyoxyethylene-polyoxypropylene block copolymer, also known as poloxamer, is sold under the trademark Pluronic ^{™}. (BASF, Mt. Olive, NJ) The preferred grade of Pluronic^{™} Poloxamer is F-127 (poloxamer 407).

As another alternative, the gelling agent is a hygroscopic high molecular weight polymer. Hygroscopic high molecular weight polymers include, for example, cellulose derivatives such as methylcellulose, hydroxyethyl cellulose, or hydroxypropyl cellulose (HPC); anhydrous maleic acid-methylmethacrylate copolymers or esters thereof (*e*.*g*., methylesters and ethylesters); polyvinylpyrrolidine or derivatives thereof (*e*.*g*., N-methylvinylpyrrolidine); vinyl-acetate; polyvinyl-alcohol; or a combination thereof. In a preferred embodiment, the gel contains from about 1% to about 5% w/w of a cellulose derivative, for example, hydroxypropyl cellulose (HPC). HPC has molecular weight of about 370,000 to 1,150,000 D. Preferably, the cellulose derivative is present in the composition in a concentration at about 1.50% w/w.

The pharmaceutical composition may contain a combination of the α1-antitrypsin and one or more additional pharmaceutically active agents. Pharmaceutically active agents useful in the composition include, without limitation, corticosteroids such as, for example, hydroxytriamcinolone, alpha methyl dexamethasone, dexamethasone acetate, betamethasone, beclomethasone dipropionate, betamethasone benzoate, betamethasone dipropionate, betamethasone valerate, clobetasol valerate, clobetasol propionate, desonide, desoxymethasone, dexamethasone, difluorosone diacetate, diflucortolone valerate, fluadrenolone, fluclorolone acetonide, flumethasone pivalate, fluocinolone acetonide, fluocinonide, flucortine butylester, flucortolone, fluprednidine (fluprednylidene) acetate, flurandrenolone, halcinonide, hydrocortisone acetate, hydrocortisone butyrate, hydrocortisone valerate, 11-desoxycortisol, methylprednisolone, triamcinolone, triamcinolone acetonide, triamcinolone diacetate, triamcinolone hexacetonide, cortisone, cortodoxone, flucetonide, fludrocortisone, difluorosone diacetate, fluradrenolene acetonide, medrysone, amcinafel, amcinafide, betamethasone and the balance of its esters, chloroprednisone, clocortelone, clocortelone pivalate, clescinolone, dichlorisone, difluprednate, flucloronide, flunisolide, fluoromethalone, fluperolone, fluprednisolone, hydrocortisone, meprednisone, paramethasone, paramethasone acetate, prednisolone, prednisolone acetate, prednisolone tebutate, prednisone, beclomethasone dipropionate, alclometasone dipropionate, mometasone furoate, or combinations thereof.

Other pharmaceutically active agents include, for example, antibiotics, cytotoxic drugs, antivirals, anti-inflammatory drugs (*i*.*e*., salicylates, colchicine, para-aminophenol, propionic acid, piroxicam, ketorolac, ketoprofen, cyclooxygenase type II inhibitors and indomethacin, among others), antihelmintics, hormones, growth factors, vitamins, antineoplastic agents, immune-response agents, immunosuppressive agents (*i*.*e*., FK506, tacrolimus, pimecrolimus, among others), antithrombotics, sulfones, sunscreens, local anesthetics (*i*.*e*., proparacaine), muscle relaxants, blood regulators, anticoagulants, hemostatics, sedatives, analgesics, adrenergics, antispasmodics, bone-active agents, prostaglandins, anorexigenics, cholinergics, anticholinergics, sulfonamides, or a combination thereof, among others.

In one embodiment, the pharmaceutical composition contains an antibiotic. A preferred antiobiotic includes, macrolid antibiotics, penicillins, tetracyclins, cephalosporins, quinolones, fluoroquinolones, neomycin, gentamycin, vancomycin, or a combination thereof

Clinically, macrolide antibiotics are used principally for treating infections with *Streptococci, Staphylococci*, and *Pneumococci*. Generally the toxicity of macrolide antibiotics is low. Esters of macrolide antibiotics have become therapeutically important because they result rapidly in higher blood levels, and further they are practically free of odor and are highly stable. Macrolide antibiotics are classified according to the size of the macrocyclic lactone ring. Macrolide antibiotics are polyfunctional molecules, most of which have at least one amine sugar and are basic.

Suitable macrolide antibiotics include those with 12-member lactone rings such as methymycin and neomethymycin. Also included are macrolide antibiotics with 14-member lactone rings, of which the preferred representatives are the erythromycins, produced from *Streptomyces erythreus*. Examples include, erythromycin A, erythromycin B, erythromycin C, erythromycin D, erythromycin E, erythromycin estolate, erythronolid, and clarythromycin. Other examples of macrolide antibiotics with 14-member lactone rings include, megalomycin and its derivatives, picromycin, narbomycin, oleandomycin, triacetyl-oleandomycin; and the neutral compounds laukamycin, kujimycin A, albocyclin, and cineromycin B. Macrolide antibiotics having 16-member rings include, carbomycin (Magnamycin) and its derivatives (*i*.*e*. niddamycin), spiramycin and its derivatives, leucomycin and its derivatives (*i*.*e*. midecamycin, maridomycin, tylosin, cirramycin, and juvenimicins); and the neutral representatives chalcomycin and neutramycin. Examples of macrolide antibiotics with larger lactone rings, *i*.*e*. having 26-40 or more ring members, include pimaricin, lucensomycin, nystatin, amphotericin B, hamycin, candicidin A and B, candidin, and levorin. The effectiveness of this group is practically exclusively against fungi and yeasts.

The composition may contain a toxin or a cytotoxic agent, as a pharmaceutically active agent, in combination with the α1-antitrypsin. Such a composition is used for specific destruction of cells (*i*.*e*., the destruction of tumor cells) by administering the protease inhibitor in association with toxins or cytotoxic prodrugs.

As used herein, the term "toxin" refers to compounds that bind and activate endogenous cytotoxic effector systems, radioisotopes, holotoxins, modified toxins, catalytic subunits of toxins, or a combination thereof, among others. Toxins that may be used according to the methods described herein include, but are not limited to, radioisotopes known in the art, compounds such as, for example, antibodies (or a complement fixing portion thereof) that bind an inherent or induced endogenous cytotoxic effector system, thymidine kinase, endonuclease, RNAse, alphe toxin, ricin, abrin, pseudomonas exotoxin A, diphtheria toxin, saporin, momordin, gelonin, pokeweed antiviral protein, alpha-sarcin, cholera toxin, or a combination thereof, among others.

As used herein, the term "cytotoxic prodrug" refers to a non-toxic compound that is converted by an enzyme, normally present in the cell, into a cytotoxic compound. Cytotoxic prodrugs that may be used in the composition described herein includes, but are not limited to, glutamyl derivatives of benzoic acid mustard alkylating agent, phosphate derivatives of mitomycin C, cytosine arabinoside, doxorubicin, and phenoxyacetamide derivatives of doxorubicin.

The pharmaceutical composition optionally contains additional compounds that assist in maintenance, storage, stabilization, cosmetic applications, or serve to prevent aggregation. These compounds include, without limitations, preservatives, reducing agents (*i*.*e*., dithiothreitol, N-acetylcysteine, 2-mercaptoethanol, cysteine, glutathione), antioxidant agents (*i*.*e*., ascorbic acid, methionine), metal chelating agents (*i*.*e*., EDTA, citrate), bulking agents/stabilizers (*i*.*e*., trehalose, glycine, mannitol, dextrans, sorbitol, glycerol, propylene glycol, albumin, disaccharides such as sucrose, cyclic oligosaccharides such as cyclodextrins, L-ascorbic acid or its derivatives, tocopherol, or a combination thereof, among others), surfactants (*i*.*e*., tween, nonidet, triton, (TRITON X-114 (polyethylene glycol tertiary octylphenyl ether), TRITON X-100 (polyethylene glycol mono [p-(1,1,3,3-tetramethyl-butyl) phenyl] ether)), and/or span; dyes, excipients; perfumes, fragrances, opacifiers, or a combination thereof. Such materials, when added, should not unduly interfere-with the activity of the pharmaceutically active drug or agents nor should they possess irritating properties.

Preservatives are optionally included in the composition described herein to maintain the integrity of the composition. It is known that formulations containing an aqueous phase in combination with a protein are susceptible to attack by bacteria and fungi. Microbial growth not only contaminates the formulation but is a potential toxicity hazard and a source of infection for patients. It is especially important to minimize microbial growth in topical formulations applied to broken or inflamed skin. Viscosity degradation reported with some polymers when exposed to microbial contamination is also of concern. Preservatives useful in the composition include, for example, without limitation, quaternium, methylparaben, phenol, para-hydroxybenzoate compounds, propyleneglycol, propylparaben, or a combination thereof.

Stabilizers and/or bulking agents are agents that help to preserve biological activities on a long-term basis and also improve the water solubility of a protease inhibitor. Accordingly, in a preferred embodiment, the composition contains a stabilizer/bulking agent such as, for example, albumin from about 0.01 % to 5% w/w, and preferably from about 0.1 % to about 1% w/w; sucrose from about 0.5% to about 30.0% w/w, and preferably from about 1.0% to about 10.0% w/w; or cyclodextrin from about 1.0% to about 10% w/w, and preferably from about 2.0% to about 5.0% w/w.

Optionally, the composition described herein additionally contains agents including, for example, an HMG-CoA reductase inhibitor. HMG-CoA reductase inhibitors suitable for use in the composition and method described herein include, but are not limited to, mevastatin, lovastatin, fluvastatin, pravastatin, sirnvastatin, dalvastatin, cerivastatin and atorvastatin, oxysterols, and 25-hydroxycholesterol, among others.

In order to treat or prevent ichthyosis, the composition described herein is administered to a subject in need of treatment or desiring prevention of the disorder. The subject is, for example, a mammal, including a human or an animal.

A gel formulation as described herein may be administered by swabbing, brushing or otherwise coating a site of injury or a wound with the gel. The composition is optionally prepared with a muco adhesive polymer that binds to the site of injury.

Topical therapy may be useful prophylactically in areas that are known to have a high probability of inducing an inflammatory response. In these instances, the treatment may be instituted immediately to help prevent subsequent complications.

Alternatively, the composition is delivered in a controlled release system. In one embodiment, a pump may be used (see, Sefton, Biomed. Eng. 14:201 (1987)). Osmotic mini-pumps may also be used to provide controlled delivery of high concentrations of the composition through cannulae to the site of interest. In another embodiment, polymeric materials can be used (*see*, Ranger and Peppas, J., Macromol. Sci. Rev. Macromol. Chem. 23:61 (1983); and Levy et al., Science 228:190 (1985)). In yet another embodiment, a controlled release system can be placed in proximity of the therapeutic target, *i*.*e*., the lung. Additional controlled release systems known to those skilled in the art are discussed in a review article by Langer, Science 249:1527-1533 (1990). Furthermore, the composition described herein may be incorporated into biodegradable polymers (*i*.*e*., encapsulation in liposomes, microparticles, and microcapsules, among others) allowing for sustained release of the compound. The polymers are being implanted in the area of the body where the drug delivery is desired, for example, at the site of a tumor or an injury so that the composition is slowly released systemically. Biodegradable polymers and their use are described, for example, in detail in Brem et al., J. Neurosurg. 74:441-446 (1991), which is hereby incorporated by reference in its entirety. Within other embodiments, the composition may be placed in any location such that the compound is continuously released into the aqueous humor. Administration can be systemic or local.

The composition may conveniently be presented in unit dosage form prepared by conventional pharmaceutical techniques. Such techniques include the steps of bringing into association the active ingredient and the pharmaceutical carrier(s) or excipient(s). In general, the formulations are prepared by uniformly and intimately bringing into association the active ingredient with liquid carriers or finely divided solid carriers or both, and then, if necessary, shaping the product In another embodiment, the composition is formulated for parenteral applications administration. Formulations suitable for parenteral administration include aqueous and non-aqueous sterile injection solutions. These solutions contain anti-oxidants, buffers, bacteriostats and solutes that render the formulation isotonic with the blood of the intended recipient. Additionally, the formulation contains aqueous and non-aqueous sterile suspensions including suspending agents, thickening agents, or both, among others. Where necessary, the composition may also include a solubilizing agent and a local anesthetic such as lignocaine to ease pain at the site of administration.

Generally, the ingredients are supplied either separately or mixed together in unit dosage form, for example, as a dry lyophilized powder or water free concentrate in a hermetically sealed container such as an ampoule or sachette indicating the quantity of active agent Where the composition is to be administered by infusion, it can be dispensed with an infusion bottle containing sterile pharmaceutical grade water or saline.
Where the composition is administered by injection, an ampoule of sterile water for injection or saline can be provided so that the ingredients may be mixed prior to administration.

The composition may be presented in unit-dose or multi-dose containers, for example, sealed ampoules and vials, and may be stored in a freeze-dried (lyophilized) condition requiring only the addition of the sterile liquid carrier, for example, water for injections, immediately prior to use. Topical formulations may be prepared from sterile powders, granules and tablets of the kind previously described.

Alternatively, the composition is administered orally. Oral formulations include standard carriers such as pharmaceutical grades of mannitol, lactose, starch, magnesium stearate, sodium saccharine, cellulose, magnesium carbonate, sucrose, trehalose, etc. Examples of suitable pharmaceutical carriers are described in "Remington's Pharmaceutical Sciences" by E.W. Martin. Such compositions will contain a therapeutically effective amount of the compound, preferably in purified form, together with a suitable amount of carrier so as to provide the form for proper administration to the patient The formulation should suit the mode of administration.

The pharmaceutically effective amount of the α1-antitrypsin that is effective in the treatment, amelioration or prevention of ichthyosis can be determined by standard clinical techniques. In addition, *in vitro* assays are employed to help identify optimal dosage ranges. In particular, the dosage of the α1-antitrypsin will depend on the disease state or condition being treated, other clinical factors such as weight and condition of the human or animal, and the route of administration of the composition. The precise dose to be employed in the formulation, therefore, should be decided according to the judgment of the practitioner and each patient's circumstances. Effective doses may be extrapolated from dose-response curves derived from *in vitro* or animal model test systems.

For treating humans or animals, a dosage between approximately 0.05 to 100 mg drug /kilogram of body weight is a typical broad range for administering the pharmaceutical composition. The methods provided herein contemplate single as well as multiple administrations, given either simultaneously or over an extended period of time. The individual dosage is determined according to the particular condition being treated. The compositions may be applied from one to six times or more daily or otherwise as is necessary to treat the skin condition. If a pharmaceutically active agent such as corticosteroid is used, then the composition is preferably applied one to four times daily, more preferably, once or twice daily. In the most preferred application, the composition is applied as a thin layer to the affected area twice daily and rubbed into the skin completely.

The pharmaceutical composition may be prepared according to a procedure that ensures suitable pH conditions within the gel, optimum α1-antitrypsin solubility, gel consistency, and sterility in the resulting product. The gel formulation is preferably intended for topical administration, and dosage forms are provided that are specially formulated for the therapeutic use of a protease inhibitor as a topical formulation. The dosage forms selected for topical applications should ideally release large amounts of the α1-antitrypsin, be sterile and non-toxic for the wound.

A preferred method of making the formulation is as follows. A powdered gelling agent is combined with an aqueous solution, such as a physiological buffer until hydrated to form a gel. The pH of the gel is adjusted to a neutral pH, preferably between approximately pH 5.5 to 9. The gel is then sterilized, preferably by irradiation. The α1-antitrypsin, preferably contained in a vehicle control buffer, is then mixed with the gel solution to produce the gel formulation. The pH of the gel is then adjusted, if necessary to a pH between approximately 5.5 and 9, preferably from about pH 6.5 to pH 8, more preferably from about pH 7 to pH 7.5, most preferably pH 7.5. A preservative is optionally added before, during or after formulation of the gel. The gel formulation is then stored under conditions that maintain sterility and activity, such as in a sealed vessel in a refrigerator or cold room.

For example, hydroxyethyl cellulose at a concentration of about 1.5% w/w is combined with 20 mM sodium phosphate at a pH of about 7.4 and stirred until fully hydrated. A recombinant alpha 1-antitrypsin (rAAT) plus a vehicle control buffer is added to the gel solution while mixing. The rAAT is normally supplied as a 5% solution in phosphate, potassium chloride, or sodium chloride, N-acetylcysteine, sodium citrate buffers at a concentration of 20 mM:200 mM:5 mM:5 mM at a pH of approximately 7.5.
If appropriate, the composition additionally contains a preservative including, for example, quaternium at a concentration of about 0.02% w/w, and a pH of about 7.5. The gel formulation is then stored in airtight tubes at 4 °C until use.

In another embodiment, the gel is lyophilized. A preferred gel formulation with desirable stability and activity is lyophilized and dissolved in water prior to use. Alternatively, stable gel formulations are lyophilized with reduced salt concentrations and reconstituted in sterile saline prior to use. The shelf life of the gel formulation prepared in accordance with the methods described herein is preferably at least one year.

### Examples

This invention is further illustrated by the following examples, including the Table, which are not to be construed in any way as imposing limitations upon the scope thereof. On the contrary; it is to be clearly understood that resort may be had to various other embodiments, modifications, and equivalents thereof which, after reading the description herein, may suggest themselves to those skilled in the art without departing from the spirit of the present invention which is defined by the scope of the appended claims.

The following Table demonstrates examples of antitrypsin gel formulations and corresponding activity and stability data.

**Table 1.Stability Summary (Series 1)**

| Purpose: | | To evaluate alpha 1-antitrypsin in different aqueous gels. | | | | |
|---|---|---|---|---|---|---|
| | | Product : Package Material: | | alpha 1-antitrypsin Gel, 0.5% Clear, glass, 4 ml short vials | | |
| | | **%w/w** | | | | |
| **Components** | | **AA-6A** | **AA-7B** | **AA-14A** | **AA-19A** | ***AA-20A** |
| **Antitrypsin (51.65 mg/ml)** | | 10.0 | 10.0 | 10.0 | 10.0 | 10.0 |
| **Propylene Glycol** | | --- | --- | --- | --- | 10.0 |
| **Glycerin** | | --- | --- | 20.0 | --- | --- |
| **Benzyl Alcohol** | | 1.0 | 1.0 | --- | --- | --- |
| **Methylparaben** | | --- | --- | --- | 0.2 | 0.2 --- |
| **Propylparaben** | | --- | --- | 0.2 | 0.04 | 0.04 |
| **Hydroxyethyl cellulose (HHX)** | | 2.0 | 2.0 | 0.04 | 0.5 | --- |
| **Carhopol 980** | | --- | --- --- | 2.0 | --- | 0.5 |
| **400 mM Phosphate/Citrate Buffer** | | --- | 25.0 | 25.0 | --- | --- |
| **NaOH, 10%** | | --- | --- | --- | pH to 7.5 Qsad | pH to 7.5 Qsad |
| **Purified Water** | | 97.0 | 62.0 | 42.76 | | |

| | | **Description** | | | | |
|---|---|---|---|---|---|---|
| **2 weeks** | | clear | Hazy | clear | clear | clear |
| **pH (1:9)** | | 7.45 | 7.71 | 7.68 | 8.06 | 7.76 |
| **1 month** | | N/t | n/t | n/t | clear | clear |
| **3 months** | | N/t | n/t | n/t | TBD | TBD |
| | | | | | | |

| | | **RAAT Activity (% Label Claim)** | | | | |
|---|---|---|---|---|---|---|
| **T=0** | | 97.2% | | | | |
| **2 weeks** | **5°C** | N/a | n/a | 46.7 | 97.7 | 105.6 |
| | **25°C** | N/a | n/a | n/a | 99.3 | 96.2 |
| | **40°C** | N/a | n/a | n/a | n/a | n/a |
| | **-20°C** | N/a | n/a | 67.1 | 98.3 | 93.2 |
| | | | | | | |
| **1 month** | **5°C** | N/t | N/t | n/t | 105.5 | 103.4 |
| | **25°C** | N/t | N/t | n/t | 102.3 | 88.3 |
| | | | | | | |
| **2 months** | **25°C** | N/t | N/t | n/t | n/t | 74.0 |
| | | | | | | |
| **3 months** | **5°C** | N/t | N/t | n/t | 107.5 | 88.5 |
| | **25°C** | N/t | N/t | n/t | 92.3 | 68.0 |
| | **25°C** | N/t | N/t | n/t | 92.3 | 68.0 |

| | | | | | | |
|---|---|---|---|---|---|---|
| Contains only 90% of targeted concentration, % w/w = 9.0%. n/a No Activity n/t Not Tested TBD To Be Determined | | | | | | |

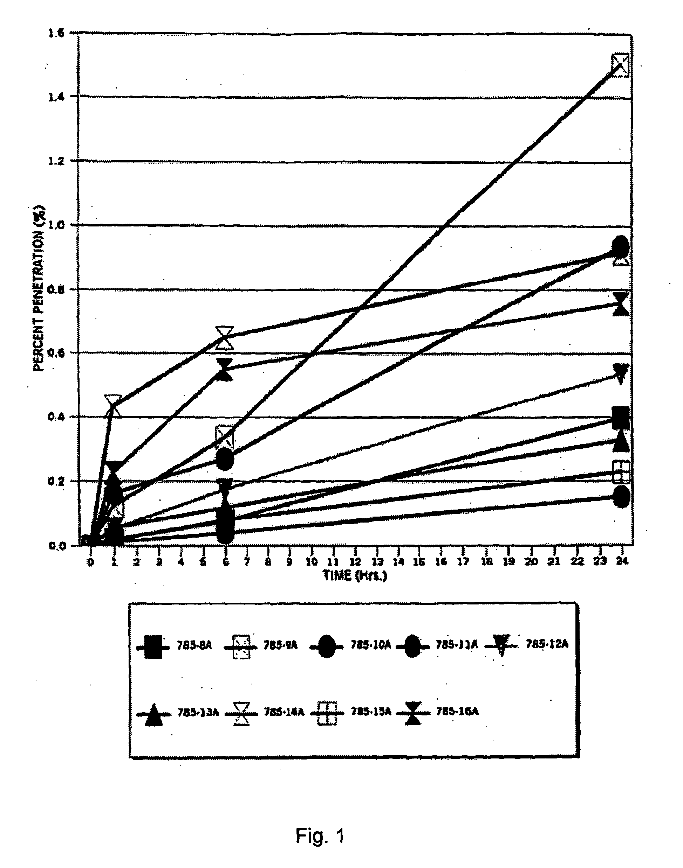

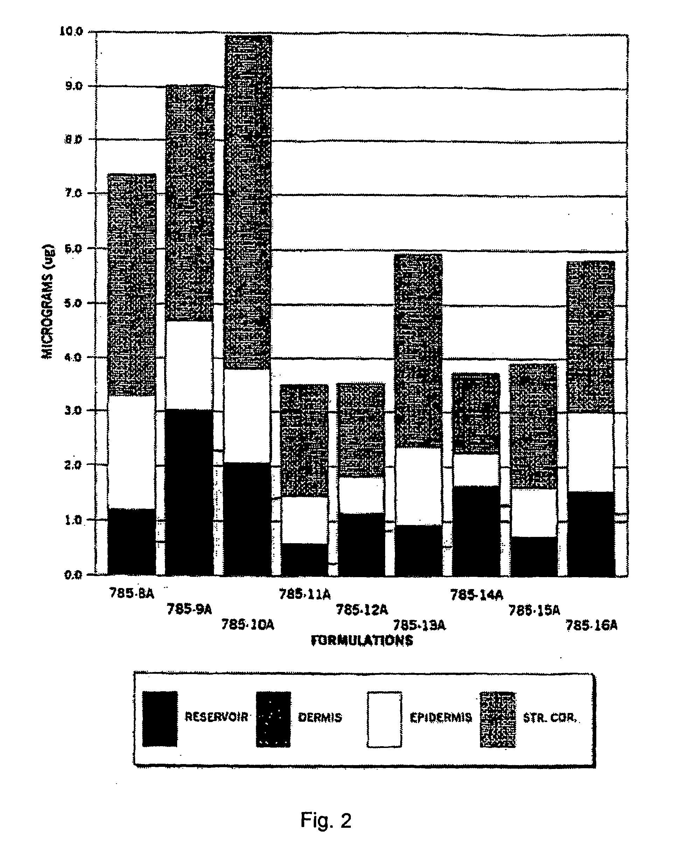

## Claims

1. Use of α1-antitrypsin and a gelling agent for the manufacture of a medicament for treating or preventing ichthyosis.

2. The use of claim 1, wherein the composition further comprises a physiological buffer at a pH of 6 to 9.

3. The use of claim 2, wherein the buffer has a pH of 6.5 to 7.5.

4. The use of any preceding claim, wherein the gelling agent is hydroxymethylcellulose, hydroxypropylcellulose, polyacrylic acid, a polyoxyethylene-polyoxypropylene block copolymer, or a combination thereof.

## Patentansprüche

1. Verwendung von α1-Antitrypsin und eines Geliermittels für die Herstellung eines Medikaments zur Behandlung oder Vermeidung von Ichthyose.

2. Verwendung nach Anspruch 1, worin die Zusammensetzung ferner einen physiologischen Puffer bei einem pH von 6 bis 9 umfasst.

3. Verwendung nach Anspruch 2, worin der Puffer einen pH von 6,5 bis 7,5 hat.

4. Verwendung nach irgendeinem vorangehenden Anspruch, worin das Geliermittel Hydroxymethylcellulose, Hydroxypropylcellulose, Polyacrylsäure, ein Polyoxyethylen-Polyoxypropylen-Blockcopolymer oder eine Kombination davon ist.

## Revendications

1. Utilisation d'α1-antitrypsine et d'un agent gélifiant pour la préparation d'un médicament pour le traitement ou la prévention de l'ichthyose.

2. Utilisation selon la revendication 1, dans laquelle la composition comprend en outre, un tampon physiologique de pH 6 à 9.

3. Utilisation selon la revendication 2, dans laquelle le tampon a un pH allant de 6,5 à 7,5.

4. Utilisation selon l'une quelconque des revendications précédentes, dans laquelle l'agent gélifiant est l'hydroxyméthylcellulose, l'hydroxypropylcellulose, le poly(acide acrylique), un copolymère séquencé poly(oxyde d'éthylène)-poly(oxyde de propylène), ou une combinaison de ceux-ci.
